Europäisches Patentamt

(19) European Patent Office

Office européen des brevets

(11) Publication number: **0 063 438**
**A1**

## (12) EUROPEAN PATENT APPLICATION

(21) Application number: **82301759.5**

(22) Date of filing: **02.04.82**

(51) Int. Cl.³: **C 12 N 1/04**
**A 23 C 9/123**

(30) Priority: **11.04.81 GB 8111446**
**11.04.81 GB 8111447**

(43) Date of publication of application:
**27.10.82 Bulletin 82/43**

(84) Designated Contracting States:
**AT BE CH DE FR GB IT LI LU NL SE**

(71) Applicant: THE SCOTTISH MILK MARKETING BOARD
Underwood Road
Paisley PA3 1TJ Scotland(GB)

(72) Inventor: Hynd, John
40 Mayfield Avenue Stranraer
Wigtonshire Schotland(GB)

(74) Representative: Szczuka, Jan Tymoteusz et al,
Cruikshank & Fairweather 19 Royal Exchange Square
Glasgow G1 3AE Scotland(GB)

(54) Production of powder containing bacteria and product thereof.

(57) This invention relates to a method of producing a substantially dry powder containing large numbers of viable bacteria which method comprises the steps of spraying a concentrated solution or suspension of viable bacteria onto a powdery material which material has not more than a limited moisture content, and drying the sprayed powder material at a low temperature and slow rate. A preferred method of the present invention provides a 'low-heat' milk-based powder suitable for use in a milk-replacer for a mammal, said powder containing a lactoperoxidase, thiocyanate and large numbers of viable bacterial capable of producing hydrogen peroxide in the stomach of a said mammal in use of said powder.

EP 0 063 438 A1

Croydon Printing Company Ltd.

## PRODUCTION OF POWDER CONTAINING BACTERIA
### AND PRODUCT THEREOF

This invention relates to a method of producing a substantially dry powder containing large numbers of viable bacteria and to a product of such a method and the use thereof.

The use of bacteria to perform various processes in industry, especially the food processing and agricultural industries, is of ever increasing importance. This has resulted in a need for products which contain large numbers of viable bacteria i.e. living bacteria able to perform their required role and preferably able to proliferate and which products can at the same time be readily handled. A preferred form, for handling purposes, of materials containing bacteria is as a substantially dry powder.

Possibly the most widely used process for producing powders from biological materials containing bacteria, e.g. yoghourt, is by means of spray drying. However even when using drying temperatures as low as $140^{\circ}$C the major part of any bacterial population is usually inactivated. Attempts have been made to circumvent this problem by producing special strains of bacteria resistant to spray-drying conditions. This approach does however entail the expenditure of considerable time and effort and it is difficult to avoid the loss at the same time of other, desirable, properties of the bacteria concerned.

It is an object of the present invention to overcome or minimize one or more of the above disadvantages.

The present invention provides a method of producing a substantially dry powder containing large numbers of viable bacteria which method comprises the steps of spraying a concentrated solution or suspension of viable bacteria onto a powdery material which material has not more than a limited moisture content, and drying the sprayed powder material at a low temperature and slow rate.

By means of the method of the present invention it is possible to obtain substantially dry powdered materials containing large numbers of viable bacteria in a relatively simple and economic manner.

As used herein "a substantially dry powder" is generally a powder having a moisture content not greater than 2% w/w, preferably not greater than 4% w/w.

The method of the invention may be practised with a wide range of powdery materials and bacterial strains for a variety of purposes. The method of the invention is however particularly applicable to powdery materials of biological material and in a preferred aspect includes as a preliminary step the formation of a powdery material having not more than a limited moisture content, preferably not more than 12% w/v. Conveniently the formation consists of or includes a spray drying step wherein an aqueous solution or suspension of the biological material is spray dried in a more or less conventional manner, for example, at from 170 to 200$^\circ$C. Since the bacteria are not subjected to this step their viability will of course not be affected by it.

Preferably there is used a solution or suspension containing not less than $0.5 \times 10^8$ bacterial cells per ml., for example, for $0.5 \times 10^8$ to $1 \times 10^{11}$ cells per ml.

In the method of the invention the sprayed powder material is preferably dried at a temperature not greater than 50$^\circ$C most preferably not greater than 35$^\circ$C. The drying rate is also desirably controlled so that a moisture content reduction of the order of from 14% w/v down to 2% w/v water is achieved in not less than 15-20 minutes. Most conveniently the drying is achieved by passing air at a temperature of from 50 to 100$^\circ$C, preferably from 50 to 70$^\circ$C through a fluidized bed of the sprayed powder material.

In another aspect the invention provides a substantially dry powder containing large numbers of viable bacteria produced by a method of the invention.

One particularly useful product which can be obtained by this invention is a bactericidal product and in particular a bactericidal product based on the lactoperoxidase system.

The lactoperoxidase system which occurs naturally in the milk of a number of mammalian species, comprises a lactoperoxidase enzyme, thiocyanate, and hydrogen peroxide. In the presence of hydrogen peroxide the enzyme converts the thiocyanate ions to an intermediate oxidation product (thought to be the hypothiocyanate ion) which is particularly bactericidal for gram negative organisms. In maternally fed calves the hydrogen peroxide is supplied by lactobacilli in the abomasum or fourth stomach of the calf.

The utility of this system in protecting young mammals against infection and improving growth rate etc. has been already recognized in the art and the desirability of incorporating it into milk substitute or milk replacer products correspondingly appreciated. Such materials are however normally produced in the form of powders (which can be made up into liquid form immediately before use) as this is the most convenient form for handling. Unfortunately there are considerable difficulties in producing such powders due to the relatively high susceptibility of lactoperoxidases and bacteria to denaturation and loss of activity. Although this has not to date been conclusively established it is believed that the denaturation may be due, at least in part, to sudden changes in osomotic pressure occurring during drying steps in the powder production process.

The present invention provides a 'low-heat' milk-based powder suitable for use in a milk-replacer for a mammal, said powder containing a lactoperoxidase, thiocyanate and large numbers of viable bacteria capable of producing hydrogen peroxide in the stomach of a said mammal in use of said powder.

As used herein the expression 'low-heat' indicates a product which is obtained under relatively mild conditions, as far as possible, including the limiting of any heating, as required for example in drying, to relatively low values.

In another aspect the invention provides a method of producing a 'low-heat' milk-based powder suitable for use in a milk-replacer for a mammal, which method comprises the steps of reducing milk or a liquid fraction thereof containing a substantial proportion of the lactoperoxidase content of milk and containing thiocyanate, to a powdery material having not more than a limited moisture content, under conditions such that the lactoperoxidase is substantially not inactivated; spraying a concentrated solution or suspension of viable bacteria capable of producing hydrogen peroxide in the stomach of a said mammal in use of the powder, onto said material; and drying the sprayed powdery material at a low temperature and a slow rate so as to form a substantially dry powder.

Thus the present invention provides a low-heat milk-based powder suitable for use in milk-replacers and incorporating a bactericidal system corresponding closely to the naturally occurring lactoperoxidase bactericidal system in milk, in a relatively simple and effective manner. By incorporating the bacteria in the powder only after most or all of the water content of milk or liquid fraction thereof has already been removed it is possible to carry out this water content removal with maximum efficiency subject to preservation of lactoperoxidase activity without adversely affecting the viability of the bacteria. By preserving the viability of the bacteria, the latter are able to proliferate in the stomach of the mammal thereby increasing still further hydrogen peroxide production and thereby bactericidal activity in the stomach.

Various milk fractions may be employed in the method of the present invention. Fresh, antibiotic-free, unpasteurised skim milk is particularly preferred. Pasteurised skim milk may also be used but is less preferred due to loss of some lactoperoxidase activity, the number of lactoperoxidase units present being of the order of only half that in unpasteurised skim milk. Preconcentrated skim milk obtained from fresh skim milk and cooled to around $5^{\circ}C$ and stored overnight will still retain acceptable levels of active lactoperoxidase. Fresh whey obtained as a by-product in cheese production or concentrated whey obtained by procedures similar to those outlined above may also be used but again is less preferred due to loss of lactoperoxidase activity - in this case to around one quarter of the number of units present in unpasteurised skim milk.

The milk or milk fraction is preferably pasteurised at a temperature of from 70 to 74°C for from 5 to 15 seconds, for example 15 seconds. At higher temperatures and longer periods significant lactoperoxidase inactivation tends to take place.

The water content of the milk or milk fractions may be reduced by various methods known for the removal of water from such compositions without affecting enzyme activity. Conveniently the milk or milk fraction is first concentrated to a total solids content of the order of 50 to 55% by, for example, evaporation or concentration in a falling film evaporator.

The concentrated liquid is then conveniently spraydried at a limited temperature such that lactoperoxidase activity is substantially unimpaired. Preferably a spray-drying is carried out with an inlet temperature of from 180 to 185°C and an outlet temperature of 80 to 90°C.

Desirably the moisture content is reduced to a value not greater than 14% w/w, preferably not greater than 10% w/w, in the powdery material thus obtained.

Various hydrogen peroxide producing bacteria may be employed in the concentrated solution or suspension used for spraying, including for example yoghourt or acidophilus bacteria. Preferably though lactobacilli, especially those strains occurring naturally in the stomach of the mammal concerned, are used. Most desirably there is used a strain selected for its high hydrogen peroxide producing capacity.

Suitable concentrated solutions or suspensions of the bacteria may be obtained by various methods known in the art for culturing and processing bacterial populations, conveniently in the case of lactobacilli, these are innoculated into skim milk or other suitable starter media and incubated at a temperature of the order of from 20 to

$40^{\circ}$C for a period of from 12 to 18 hours. Coagulation of the casein in the skim milk is desirably prevented by controlling the pH of the medium within the range 5.8 to 6.0 . Conveniently control is effected by the addition of alkali. This has the advantage of improving the number of viable cells obtained by from 2 to 3 time as compared with cultrues where pH control is not used. Preferably there is used in the method of the invention a solution or suspension containing not less than $0.5 \times 10^{8}$ bacterial cells per ml., for example, from $0.5 \times 10^{8}$ to $1 \times 10^{11}$ cells per ml. Such concentrations can be readily achieved with the above referred to culturing of lactobacilli in skim milk. By maximising the concentration of the bacterial cells the amount of solution or suspension required to be sprayed onto the powdery material to achieve a desired number of viable bacteria per gm. of powder is minimized thereby minimizing the extent of drying required and thus the intensity and length of the drying process employed.

The spraying of the bacterial cell containing liquid is desirably carried out so as to achieve as far as possible a uniform admixing of the bacterial cells and the powdery material. Most conveniently this is effected by spraying onto a fluidised bed of the powdery material having a limited depth, preferably not greater than 10 cms.

In the method of the invention the sprayed powdery material is preferably dried at a temperature not greater than $50^{\circ}$C most preferably not greater than $35^{\circ}$C. The drying rate is also desirably controlled so that a moisture content reduction of the order of from 14% w/v down to 2% w/v water is achieved in not less than 15-20 minutes. Most conveniently the drying is achieved by passing air at a temperature of from 50 to $100^{\circ}$C, preferably from 50 to $70^{\circ}$C through a fluidized bed of the sprayed powder material.

Conveniently the method of the invention is carried out on a more or less continuous basis in the sense that the bacterial solution or supension is sprayed onto the powdery material substantially directly after completion of the water content reduction step, and drying of the sprayed material carried out immediately. In this way since the sprayed material will usually already be at an elevated temperature, the need for further heating will be minimized.

In general the naturally occurring thiocyanate content of milk or milk fractions of the type referred to above is sufficient to provide an acceptable bactericidal activity of the final product in use thereof. If necessary though the method may include the addition of one or more thiocyanates, conveniently alkali or alkaline earth metal or ammonium thiocyanates e.g. sodium or potassium thiocyanate to the milk or milk fraction or even to the powdery material or final substantially dry powder.

In another aspect the invention provides a substantially dry 'low-heat' milk-based powder with bactericidal activity when produced by a method of the invention.

In addition to the abovementioned advantages, the present invention provides the further advantage that by virtue of the relatively mild conditions employed in the method of the invention the formation of sulfhydryl linkages which occurs during drying at higher temperatures is largely avoided. This is beneficial as these linkages tend to inhibit the bactericidal activity of the lactoperoxidase system.

Whilst the above product of the invention itself may be used as a milk replacer (i.e. substitute for natural maternal milk) or milk substitute, it is in general desirable, especially in the case of relatively young mammals to supplement it with other feedstuff materials such as starch and glucose as well as for example minor constituents such as vitamins, minerals and/or trace elements, especially where skim milk or the like is used to produce the powder. Since this involves the loss of the animal fat present in the whole milk this is desirably replaced by vegetable and/or other fats by admixing the powder with fat-filled extender containing for example 40% vegetable and/or other fat made up for example of tallow and coconut oil in the proportions 80:20. The powder is conveniently made up with extender in the ratio 40:60.

Thus in a further aspect the invention provides a milk replacer powder comprising a substantially dry 'low-heat' milk-based powder with bactericidal activity of the invention in intimate admixture with an added feedstuff as well as a milk replacer comprising a milk replacer powder of the invention made up into an aqueous solution or suspension.

In still further aspects the present invention provides a method for the treatment or prophylaxis of gastro-intestinal bacterial infections in mammals comprising the oral administration of a product of the present invention; and a method of improving growth and/or food utilization in mammals comprising feeding said mammals with a milk-based powder, preferably a milk replacer, of the present invention. Bacterial infections that may be treated include those due to Kaerogenes, P. fluorescens and S. typhimurium bacteria as well as E. coli bacteria.

Further preferred features and advantages of the invention will appear from the following Examples given by way of illustration of the invention.

### Example 1.

A. Fresh, antibiotic-free, unpasteurized skim cows milk (10l.) is pasteurized at a temperature of $72^{\circ}C$ for 15 seconds. The pasteurized skim milk thus obtained was concentrated up to 50% total solids by concentration in a falling film evaporator. The liquid concentrate was then spray dried at an inlet temperature of 80 to $90^{\circ}C$ and a residence time of 60 seconds in a Niro spray drier (Niro Atomiser Ltd., Copenhagen, Denmark), to produce a powdery material having a moisture content of about 8% w/w.

B. An aqueous suspension of a hydrogen peroxide producing lactobacillus (Strain LB 10) which was originally obtained from the abomasum of a suckling calf (5 mils containing $4 \times 10^{8}$ cells per ml) was inocculated into skim cows milk (500 mls) and incubated at $37^{\circ}C$ for 12 hours. The pH was maintained in the range pH 5.8 to pH 6.0 by the addition of 20% aqueous ammonia using automatic titration and agitation apparatus. The resulting mixture contained $0.4 \times 10^8$ cells/ml.

C. The above mixture was sprayed onto a fluidized bed of the powdery material obtained from the spray drier at a rate of approximately 9 mls. of liquid bacterial cell containing mixture per 600 g. of the powder material.

D. The sprayed powdery material is passed through a fluidized bed drier using an air temperature of $65^{\circ}C$ and a residence time of 15 to 20 minutes to obtain a final powder having a moisture content of 3% w/w and a well distributed intimately admixed bacterial cell content. Following immediately after reconstitution of the final powder with water at ambient temperature so as to produce a 10% w/v (with respect to powder content) liquid product, the liquid product was found to contain about $2 \times 10^4$ viable lactobacillus cells per ml. This corresponds to an approximate viable lactobacillus cell content in the powder of around $2 \times 10^5$ cells per g.

As will be noted from the above the reconstitution is carried out at a relatively low temperatue in order to avoid loss of bacterial cell viability as well as to avoid the formation of sulfhydryl linkages which can inhibit the lactoperoxidase system. In order to avoid inhibition of the system it is also desirable to use antibiotic free materials to avoid loss of hydrogen peroxide producing bacteria.

Although very low moisture levels can be tolerated reasonably well by certain strains of bacteria, it is in general preferred that the drying of the sprayed powdery material is not taken to completion. Desirably the drying of the sprayed powdery material is carried out to obtain a final water content of not less than 2% w/w.

It will be appreciated that although it is in general desirable that the products of the present invention be formulated so as to provide levels of the lactoperoxidase system constituents in the reconstituted (i.e. liquid) milk replacer fed to the mammalian young corresponding generally to the levels obtaining in maternal milk and provide levels in the stomach corresponding to naturally occurring levels, the levels can be readily varied and in particular may be made slightly higher in order to obtain optimum bactericidal activity.    In general the product is desirably formulated so as to provide 1 mg (50U) of lactoperoxidase per litre of digestive juice in the stomach with 0.1 mM thiocyanate (calculated as NaSCN) and sufficient bacterial cells to produce 0.2 to 0.4mM hydrogen peroxide.    Since different bacterial strains produce hydrogen peroxide at different rates, the optimum amount of bacterial cells required will be dependent on the particular strain(s) used.

Having regard to the above the corresponding levels in a dried sprayed powder of the invention intended to constitute 40% w/w of a final milk replacer powder are desirably of the order of from 200 to 5000 p.p.m. of thiocyanate calculated as NaSCN, from 15 to 300 mg of lactoperoxidase per kg of the powder, and from $0.5 \times 10^4$ to $3 \times 10^8$ viable hydrogen peroxide producing bacterial cells per gram of the powder.

### Example 2.

A.    A yoghourt sample containing lactobacillus vulgaricus and streptococcus thermophilus (5 mls containing $5 \times 10^8$ cells/ml) was inoculated into skim cows milk (500 mls) and incubated at $40^{\circ}$C for 12 hours.    The pH of the mixture was maintained in the range pH 5.8 to pH 6.0 by the addition of 20% aqueous ammonia using automatic titration and agitation apparatus.    The resulting mixture contained $0.5 \times 10^8$ cells/ml.

B.    The above mixture was sprayed onto a fluidized bed of a low fat milk powder ('instant type i.e. of the rapidly dissolving commercially available type) at an approximate rate of 1 ml of mixture per 60 g. of milk powder.

C.    The sprayed milk powder is passed through a fluidized bed drier using an air temperature of 65$^{\circ}$C and a residence time of 15 to 20 minutes to obtain a final powder having a moisture content of 2% w/w.    Immediately after reconstitution of the powder with water at ambient temperature to produce a 10% w/v (with respect to the powder) reconstituted yoghourt, the yoghourt was found to contain approximately 5x10$^4$ viable bacterial cells per ml corresponding to around 5x10$^5$ cells per g. of the powder.

Naturally care should be taken in the subsequent use of a powder of the invention, e.g. when reconstituting it into a liquid form, not to subject it to any conditions which would adversely affect the carefully preserved viability of the bacterial cell content thereof.

Although very low moisture levels can be tolerated reasonably well by at least some bacteria, it is in general preferred that the drying of the sprayed powdery material is not taken to completion.    Desirably drying is carried out so as to retain a final moisture content of not less than 2% w/w in the final powder.

CLAIMS:

1.    A method of producing a substantially dry powder containing large numbers of viable bacteria which method comprises the steps of spraying a concentrated solution or suspension of viable bacteria onto a powdery material which material has not more than a limited moisture content, and drying the sprayed powder material at a low temperature and slow rate.

2.    A method as claimed in claim 1 which includes as a preliminary step the formation of a powdery material having not more than a limited moisture content.

3.    A method as claimed in claim 2 wherein said preliminary step comprises a spray drying step.

4.    A method as claimed in any one of claims 1 to 3 wherein the sprayed powdery material is dried at a temperature not greater than 50°C.

5.    A method as claimed in claim 4 wherein the sprayed powdery material is dried at a temperature not greater than 35°C a rate such as to produce a moisture content reduction of the order of from 14% w/v down to 2% w/v water in not less than from 15 to 20 minutes.

6.    A method of producing a 'low-heat' milk-based powder suitable for use in a milk-replacer for a mammal according to any one of claims 1 to 5, which method comprises the steps of reducing milk or a liquid fraction thereof containing a substantial proportion of the lactoperoxidase content of milk and containing thiocyanate, to a powdery material having not more than a limited moisture content, under conditions such that the lactoperoxidase is substantially not inactivated;  spraying a concentrated solution or suspension of viable bacteria capable of producing hydrogen

0063438

peroxide in the stomach of a said mammal in use of the powder, onto said material; and drying the sprayed powdery material at a low temperature and a slow rate so as to form a substantially dry powder.

7. A method as claimed in claim 6 wherein are used bacteria selected from lactobacillus, yoghourt and acidophilus bacteria.

8. A 'low-heat' milk-based powder suitable for use in a milk-replacer for a mammal, said powder containing a lactoperoxidase, thiocyanate and large numbers of viable bacterial capable of producing hydrogen peroxide in the stomach of a said mammal in use of said powder.

9. A milk replacer powder comprising a substantially dry 'low-heat' milk-based powder with bactericidal activity according to claim 8 in intimate admixture with an added feedstuff as well as a milk replacer comprising a milk replacer powder of the invention made up into an aqueous solution or suspension.

10. A method of improving growth and/or food utilization in mammals comprising feeding said mammals with a milk-based powder according to claim 8.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | DE-C- 462 254 (H.LEGER) *Claim; page 1, line 67 - page 2, line 36; page 1, lines 1-12* | 1 | C 12 N 1/04<br>A 23 C 9/123 |
| | --- | | |
| A | US-A-2 738 273 (M.MUHRER) *Claim 1; column2 ,line 52 - column 3, line 37* | 1,4,6, 7,8,9, 10 | |
| | --- | | |
| A | US-A-3 272 633 (F.CLICKNER) | | |
| | --- | | |
| A | US-A-3 914 438 (L.HOLT) | | |
| | --- | | |
| A | FR-A-1 313 073 (H.GRIFFON et al.) | | **TECHNICAL FIELDS SEARCHED (Int. Cl. 3)** |
| | --- | | |
| A | GB-A-1 134 206 (WERNER-GREN MEDICAL LABORATORY) | | C 23 C 9/00<br>C 12 N 1/00<br>A 23 K 1/00<br>A 23 C 21/00<br>A 23 L 1/00<br>A 23 C 1/00 |
| | ----- | | |

The present search report has been drawn up for all claims

| Place of search<br>THE HAGUE | Date of completion of the search<br>20-07-1982 | Examiner<br>DESMEDT G.R.A. |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82